# EUROPEAN PATENT APPLICATION

(11) **EP 2 991 008 A1**
(43) Date of publication of application: **02.03.2016**
(21) Application number: 15183063.5
(22) Date of filing: 28.08.2015
(51) Int. Cl.: G06Q 10/06, G06Q 50/22

(54) **AUTOMATED HOSPITAL WORKFORCE SYSTEM FOR LOAD DRIVEN SCHEDULING OPTIMIZATION**

(30) Priority: 29.08.2014 US 201462043560 P
(71) Applicant: Teletracking Technologies, Inc., Pittsburgh, PA 15222 (US)
(72) Inventor: DUBE, Christopher, Pittsburgh, PA 15237 (US); FLETCHER, Rodger, Gibsonia, PA 15044 (US); HARBER, Jason, Pittsburgh, PA 15237 (US); MANCINE, Nathan, Cranberry Twp, PA 16066 (US); McCLEEREY, Michelle, Lakeville, MN 55044 (US)
(74) Representative: Lucas, Brian Ronald

(57) **Abstract**

An embodiment provides a system for automated hospital workforce load driven scheduling optimization, including: a processor; and a memory storing instructions executable by the processor to: access scheduling information for a hospital unit comprising a number of staff available for working in the hospital unit and a staffing ratio for the hospital unit; access a projected census value calculated reflecting current and future patient movement and progression within and between units; calculate a number of projected workers needed for the hospital unit based on the projected census value and the staffing ratio for the hospital unit; and output the number of projected workers needed for the hospital unit. Other embodiments are described and claimed.

## Description

### CROSS REFERENCE TO RELATED APPLICATIONS

This application claims the benefit of U.S. Provisional Patent Application Serial No. 62/043,560, filed on August 29, 2014, the contents of which are incorporated by reference herein.

### BACKGROUND

Current workforce scheduling systems allow health systems to assign their workforce to specific areas of the facility using many variables, one of which is the current census in that area. Within a health system, however, the census is a rather dynamic value that continually changes, e.g., as patients are admitted, transferred, or discharged. As the "official notification" of an admission, transfer, and discharge takes place after the physical move, currently, census information is static and is based on occupied beds at the time of its calculation.

As such, supervisors and/or centralized staffing coordinators do not have adequate data available regarding upcoming staffing needs and are left to guess what level of staffing might be adequate.

### BRIEF SUMMARY

In summary, one aspect provides a system for automated hospital workforce load driven scheduling optimization, comprising: a processor; and a memory storing instructions executable by the processor to: access scheduling information for a hospital unit comprising a number of staff available for working in the hospital unit and a staffing ratio for the hospital unit; access a projected census value calculated reflecting current and future patient movement and progression within and between units; calculate a number of projected workers needed for the hospital unit based on the projected census value and the staffing ratio for the hospital unit; and output the number of projected workers needed for the hospital unit.

Another aspect provides a method for automated hospital workforce load driven scheduling optimization, comprising: accessing scheduling information, stored in an electronic memory device, for a hospital unit comprising a number of staff available for working in the hospital unit and a staffing ratio for the hospital unit; accessing, using a processor, a projected census value calculated reflecting current and future patient movement and progression within and between units; calculating, using a processor, a number of projected workers needed for the hospital unit based on the projected census value and the staffing ratio for the hospital unit; and outputting, using a processor, the number of projected workers needed for the hospital unit.

A further aspect provides a program product for automated hospital workforce load driven scheduling optimization, comprising: a device readable storage unit having code stored therewith, the code being executable by a processor and comprising: code that accesses scheduling information for a hospital unit comprising a number of staff available for working in the hospital unit and a staffing ratio for the hospital unit; code that accesses a projected census value calculated reflecting current and future patient movement and progression within and between units; code that calculates a number of projected workers needed for the hospital unit based on the projected census value and the staffing ratio for the hospital unit; and code that outputs the number of projected workers needed for the hospital unit.

The foregoing is a summary and thus may contain simplifications, generalizations, and omissions of detail; consequently, those skilled in the art will appreciate that the summary is illustrative only and is not intended to be in any way limiting.

For a better understanding of the embodiments, together with other and further features and advantages thereof, reference is made to the following description, taken in conjunction with the accompanying drawings. The scope of the invention will be pointed out in the appended claims.

### BRIEF DESCRIPTION OF THE DRAWINGS

FIG. 1 illustrates an example overview of a hospital workforce scheduling system.
FIG. 2 illustrates an example system for automated hospital workforce load driven scheduling optimization.
FIG. 3 illustrates an overview of integrating census projections and available staffing for balanced staffing plans.
FIG. 4 illustrates an example method for automated hospital workforce load driven scheduling optimization.
FIG. 5 illustrates an example method for automated bed assignment using a system for automated hospital workforce load driven scheduling optimization.

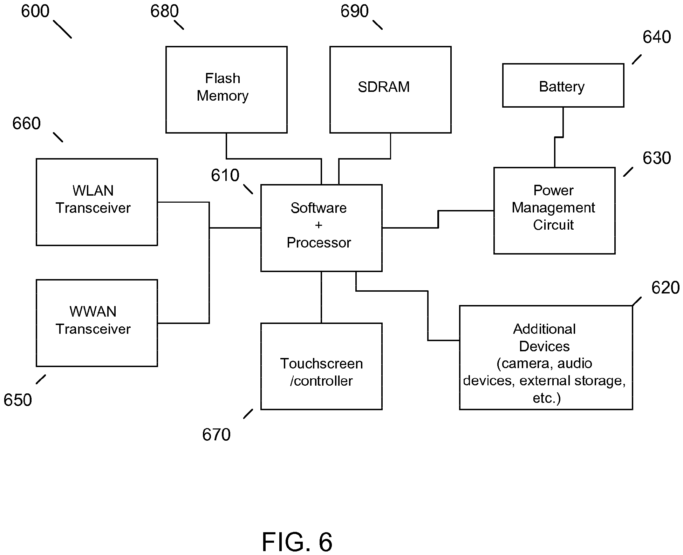

FIG. 6 illustrates an example of computer circuitry that may be used in implementing a system for automated hospital workforce load driven scheduling optimization.

### DETAILED DESCRIPTION

It will be readily understood that the components of the embodiments, as generally described and illustrated in the figures herein, may be arranged and designed in a wide variety of different configurations in addition to the described example embodiments. Thus, the following more detailed description of the example embodiments, as represented in the figures, is not intended to limit the scope of the embodiments, as claimed, but is merely representative of example embodiments.

Reference throughout this specification to "one embodiment" or "an embodiment" (or the like) means that a particular feature, structure, or characteristic described in connection with the embodiment is included in at least one embodiment. Thus, the appearance of the phrases "in one embodiment" or "in an embodiment" or the like in various places throughout this specification are not necessarily all referring to the same embodiment.

Furthermore, the described features, structures, or characteristics may be combined in any suitable manner in one or more embodiments. In the following description, numerous specific details are provided to give a thorough understanding of embodiments. One skilled in the relevant art will recognize, however, that the various embodiments can be practiced without one or more of the specific details, or with other methods, components, materials, et cetera. In other instances, well known structures, materials, or operations are not shown or described in detail to avoid obfuscation.

There are currently no products that integrate forecasted or projected hospital census data with hospital staff schedules to drive resource needs across a facility in a proactive fashion. As described herein, the current methods available require employees to manually work through several data sources to balance the staffing in an attempt to meet the upcoming patient census.

Accordingly, using census data, e.g., census data compiled within TELETRACKING's CAPACITY MANAGEMENT SUITE (CMS), a forecast of a census value for each area within a facility, e.g., areas within a hospital such as an intensive care unit, a cardiac unit, surgery, etc., is available. By integrating this forecasted or projected census data with data regarding assigned (or assignable) staff for an upcoming shift and pre-established staffing ratios for each area or unit, an embodiment is able to provide a staff balancing recommendation for each area. Moreover, an embodiment is capable of auto-populating various inputs, e.g., staffed beds, such that a facility manager has a more accurate and more readily available view of capacity for a unit or an entire facility (e.g., hospital wide view). Likewise, given that an embodiment allows for automated system updates, many fields within a management system may be auto-populated, e.g., automated bed assignment recommendations may be produced. Moreover, automated notifications and communications may be issued regarding projected staffing needs.

The embodiments described herein represent a significant technological improvement in the area of hospital workforce management systems. In the state of the art, workforce management systems are not able to leverage projected census data such that workforce staffing plans are not synchronized with projected needs using the most relevant data. However, utilizing the technological improvements described herein, workforce planning may be optimized for the challenging environment of the hospital where changing events, e.g., admissions, transfers, discharges and the like, render future workforce planning particularly difficult. Having access to a projected census value, as provided by embodiments, that may be integrated into a workforce planning technique, advances a user's (e.g., hospital administrator, unit manager, etc.) ability to ensure adequate staffing is available, balancing ever changing workloads and staffing availability on an ongoing basis with reduced manual input and labor intensive involvement.

As such, supervisors and/or centralized staffing coordinators have an automated method to "align staff to demand" that is based on realistic projected census values. An embodiment supports an automated or semi-automated method for ensuring compliance to mandated staffing ratios (regardless of whom they are mandated by, e.g. government, hospital, health system, etc.) for given units or areas of a hospital. Automated designation of staffed beds enables more accurate representation of available capacity within a specific area and across the facility. Automated bed assignment also optimizes the amount of time it takes to assign a patient to a bed.

In an embodiment, using a capacity management system, every patient movement is projected in advance. That is, Bed Request Date and Time, Activated Request Date and Time, Bed Assigned Date and Time, Ready to Move Date and Time, Projected Ready to Move Date and Time, Bed Occupied Date and Time, Pending Transfer Status, Pending Discharge Status, Confirmed Discharge Status, Projected Discharge Date and Time, and actual Discharge Time data is available from the capacity management system. This gives visibility to care areas and support functions of patient movements so that a hospital may prepare for and/or facilitate the movement. As a result, these movements provide a mechanism to forecast all of the admissions, transfers, and discharges within the facility. Using the data that is collected during all of these movements, a projected census for the facility is derived. This projected census data is output and leveraged for intelligent management of work scheduling, as further described herein.

The projected census may be derived for each area of a facility, over a configurable period of time (e.g., within a 24 hour period, such as 3 hours from the current time, etc.). Using this information, along with recommended staffing levels (e.g., patient to nurse ratio) based on the type of unit, a workforce scheduling module, which contains the staff members that are scheduled or available to be scheduled for that given shift, can automatically assign each staff member to a specific area and ensure that each area is appropriately staffed based on both the current and projected census.

In addition to projecting staffing needs, the resulting data can be leveraged across the system to drive better results and visibility. For example, an embodiment automates the population of "staffed beds." A staffed bed is one that either has, or will have, a caregiver associated with it. For example, if there are 15 physical beds in a nursing unit, 4 nurses assigned to the unit, and a staffing ratio of 3:1, only 12 of the 15 physical beds would be considered "staffed." As such, if 12 beds were filled, this unit would be considered to be at maximum capacity even though there are 3 empty physical beds. This information is relevant for example when trying to convey the overall capacity of a hospital. Without an accurate account of staffed beds in CMS, the overall reported capacity of the facility will be artificially low, which decreases the facility's ability to manage their actual capacity needs. Through the integration described, because the staff members will be allocated based on the forecasted census and staffing ratios, the number of staffed beds can be automatically derived and proliferated throughout the system.

An embodiment additionally permits automated bed assignments. For example, using a similar concept as mentioned above, once the system has assigned staff members to a unit in accordance with the projected census and staffing ratios, the system will be able to determine how many empty staffed beds are available within a given unit. As such, when a new patient is in need of a bed, based on a set of criteria (e.g., level of care needed, type of bed needed, isolations, etc.) along with the availability of empty staffed beds, the system will suggest the appropriate bed(s) for the patient rather than requiring that the bed control coordinator manually search through the electronic bedboard for the best bed available.

These and other features of the claimed embodiments are further described with reference to the figures. The illustrated example embodiments will be best understood by reference to the figures. The following description is intended only by way of example, and simply illustrates certain example embodiments.

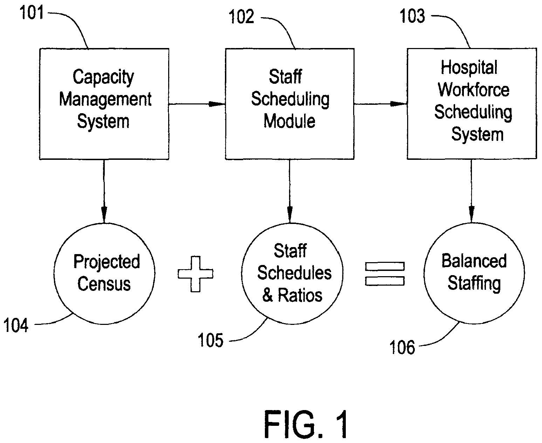

At a high-level, the integration of these data sources (projected census values, staff availability, recommended staff levels for a unit, etc.) is illustrated in FIG. 1. In the example illustration a capacity management system 101 provides a projected census value 104. For example, a projected census value 104 may be calculated using data available regarding the current status (e.g., current census) as well as projected values given impending or confirmed transfers, admissions, etc., as well as times associated therewith.

By way of specific example, a projected census value 104 may be determined by an embodiment using input data from a capacity management system including, but not necessarily limited to, a number of physical beds in a unit, a current census value, a pending discharge status, a confirmed discharge status, a pending transfer out value, a pending transfer in value, a preadmission value, as well as clean beds, dirty beds and blocked beds values. Thus, an embodiment may calculate, e.g., in three hours time, a projected census value (i.e., the number of patients expected to be in a given unit, e.g., surgery or intensive care).

A staff scheduling module 102 for its part may be configured to output appropriate staff schedules and ratios 105. For example, a predetermined staff scheduling ratio may be implemented such as one assigned nurse to three occupied beds. Given this information, the staff scheduling module 102 outputs a proposed staff schedule, e.g., one that accommodates a predetermined staffing ratio for a unit or area with a given number of beds.

In an embodiment, the actual, available staff capable of working in a unit or area during a given shift is available to a hospital workforce scheduling system 103. In an embodiment, having the projected census value for a hospital area 104, the staff schedule and ratio values for the area 105, as well as access to available staff data, a balanced staffing plan 106 may be output. This balanced staffing plan 106 accounts not only for current census, but also considers that projected census value and adjusts staff scheduling recommendations to meet these projected needs.

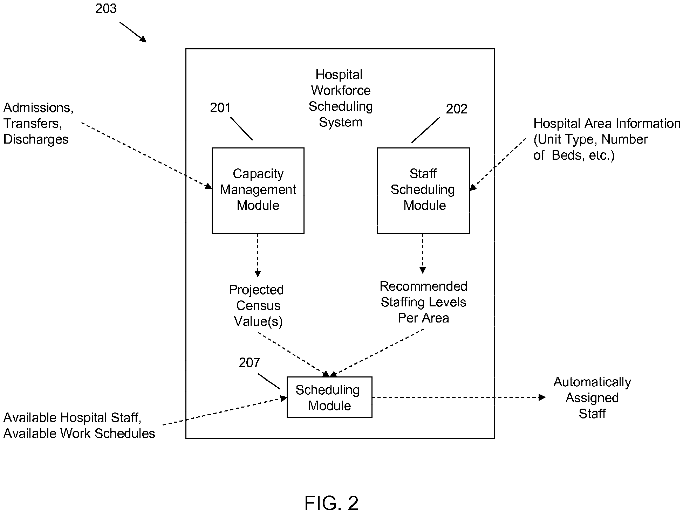

FIG. 2 illustrates an example workforce scheduling system 203 according to an example embodiment. Here, a capacity management module 201 takes as input available data used to create a projected census value, e.g., admissions, transfers, discharges, etc., as described herein. A staff scheduling module 202 takes as input available data used to create recommended staffing levels generally per area, e.g., one nurse assigned to three beds, etc. For example, hospital area information may be input to the staff scheduling module, e.g., a number of physical beds less a number of blocked beds for a particular hospital unit, as well as predetermined data regarding a staff to bed ratio, such as 3:1 by way of non-limiting example.

Given the projected census value for a hospital area and a recommended staffing level for the hospital area, as well as the physical attributes of the unit and available workers, a scheduling module 207 coordinates this information with available hospital staff and scheduling information to automatically produce a balanced staffing plan, e.g., including automatic assignment of staff to particular beds in a unit for an upcoming shift.

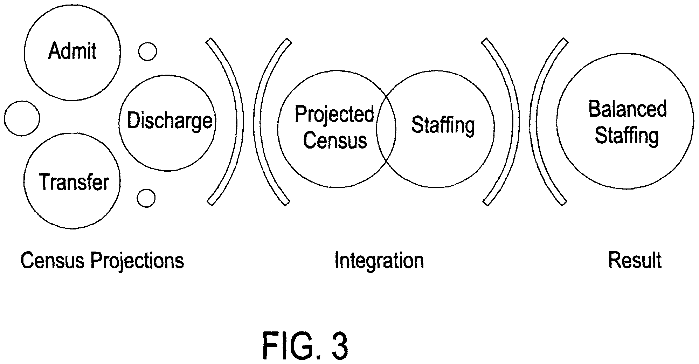

This is illustrated in a generalized view in FIG. 3. As shown, the census projections are integrated into staffing data in order to achieve balanced staffing for a future work time. Thus, census projections derived from admission, transfer and discharge data are used to match available staff to projected needs of a hospital unit or area automatically.

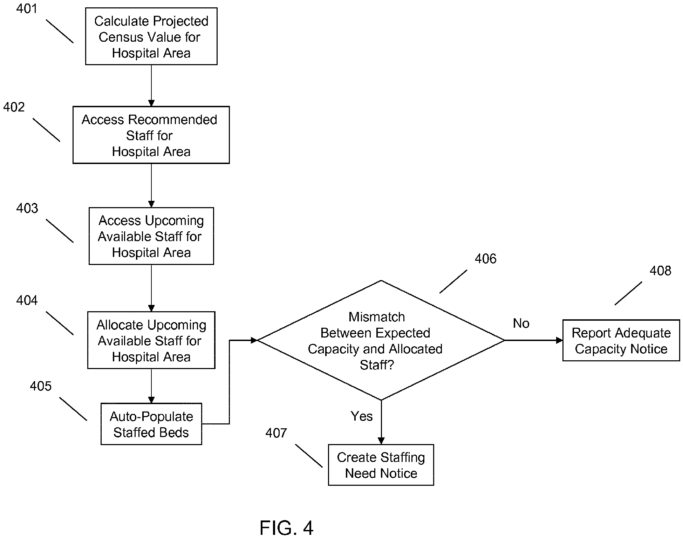

An example method of automatically producing a balanced staffing plan is illustrated in FIG. 4. In the illustrated example, an embodiment calculates a projected census value for a hospital area at 401. Again, this may include predicting an expected number of patients for the unit in question given current census data, pending discharges and transfers, expected admissions, etc.

An embodiment then accesses recommended staff for the hospital area at 402, e.g., a number of staff currently recommended for projected census value. This permits a match between projected need (represented by the projected census value) and available staff, rather than a match between a less relevant data set, e.g., physical beds in a unit and/or a current census value. Moreover, this avoids the all too common situation where a hospital fills physical beds and then reactively attempts to find staff to accommodate the filled physical beds.

The upcoming available staff for the hospital area, e.g., workers currently scheduled to work an upcoming shift within the hospital unit, is accessed at 403. This permits an embodiment to allocate the actual staff available for a shift in a unit or area of the hospital with the projected census in mind at 404. This likewise permits an embodiment to auto-populate parameters within a management system at 405, e.g., assign workers to staffed beds, identify excess staffed beds (projected empty staffed beds), etc.

Further, an embodiment provides a proactive monitoring and resolution capability. As illustrated in the example of FIG. 4, an embodiment may be utilized to determine at 406 if there exists a mismatch between an expected capacity (e.g., as represented by the projected census value for a hospital area or unit) and allocated staff (e.g., available staff scheduled to work in a hospital area or unit) as compared with a preferred or required staffing ratio, e.g., three (3) beds assigned per nurse.

If so, an embodiment may automatically generate a notification at 407, e.g., a staff need notification. This notification may be displayed locally (e.g., on a display screen operatively coupled to the system) and/or communicated to remote devices, e.g., mobile units such as smart phones of available or scheduled workers.

This permits a user, e.g., hospital area unit manager, to anticipate that the unit might be understaffed given the currently available workers scheduled to the unit or area, given the projected census value for that unit or area of the hospital. As may be appreciated, this also permits automated messaging, e.g., communications with other possible workers regarding an impending or expected need for additional capacity or workers. The converse is also true, e.g., an expected over-supply of workers or overstaffing situation may be anticipated and scheduling of workers adjusted accordingly.

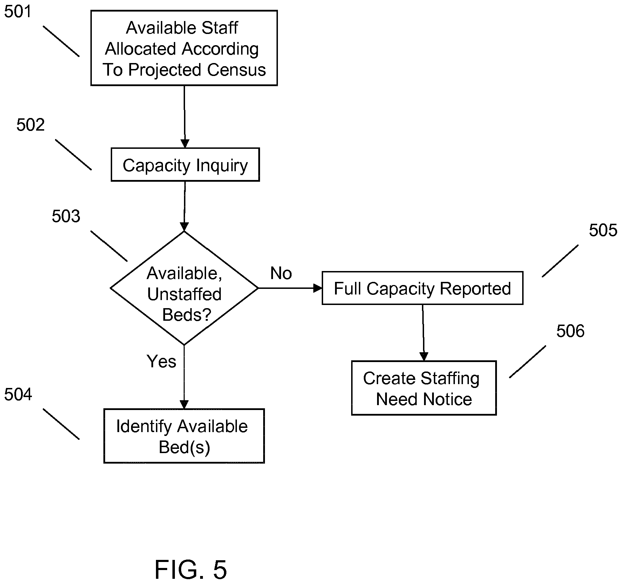

In addition, and referring to FIG. 5, an embodiment may be used to more accurately calculate projected bed assignments. This is by virtue of an embodiment producing a more realistic scheduling assessment given the projected census number.

By way of example, the available staff is allocated to an area or unit of the hospital according to the projected census number at 501, as described herein. If a capacity inquiry is received at 501, e.g., a user searches for a particular bed type, in a particular unit, etc., an embodiment may use the projected staff allocations, down to the bed level, to determine at 503 if there will be such a staffed bed available within the unit. That is, an embodiment will answer a query regarding the capacity check not simply with the data regarding physically available beds within a given unit or area, but will report whether or not a staffed bed in that unit or area will be available at the appointed time. If so, an embodiment may report back the available bed(s) at 504, e.g., bed number(s) and/or location(s). However, if a staffed bed is not projected as available, as determined at 503, an embodiment may output a full capacity notification or report at 505, as well as create a staffing need notification at 506. In this way, a user (e.g., area or unit manager) will have easy access to projected staffed bed availability within a unit in time to adjust staffing allocations.

An embodiment therefore integrates a projected census value with recommended/preferred staffing levels and data regarding actually available staff to create a balanced staffing plan. Given this forecasting availability, shortfalls and excesses regarding staff allocations can be identified early enough that remedial actions are available.

It will be appreciated that an embodiment may be implemented using a variety of types of computing devices. Such devices might take the form of a work station, a laptop computer, a hand held or mobile computer, e.g., a smart phone or tablet computing device, as well as combinations of the foregoing. The computing device(s) utilized for implementing the workforce management technology described herein may include a variety of circuitry and components.

For example, with regard to smart phone and/or tablet circuitry 600, an example includes an ARM based system (system on a chip) design, with software and processor(s) combined in a single chip 610. Internal busses and the like depend on different vendors, but essentially all the peripheral devices (620) may attach to a single chip 610. The tablet circuitry 600 combines the processor, memory control, and I/O controller hub all into a single chip 610. Also, ARM based systems 600 do not typically use SATA or PCI or LPC. Common interfaces for example include SDIO and I2C.

There are power management chip(s) 630, which manage power as supplied for example via a rechargeable battery 640, which may be recharged by a connection to a power source (not shown), and in at least one design, a single chip, such as 610, is used to supply BIOS like functionality and DRAM memory.

ARM based systems 600 typically include one or more of a WWAN transceiver 650 and a WLAN transceiver 660 for connecting to various networks, such as telecommunications networks and wireless base stations. Commonly, an ARM based system 600 will include a touch screen 670 for data input and display. ARM based systems 600 also typically include various memory devices, for example flash memory 680 and SDRAM 690. Application programs, e.g., as for example representing functionality of the capacity management module, the staff scheduling module, and/or the scheduling module, may be stored in flash memory 680 and/or SDRAM 690 for execution by processor 610. It will be, however, understood that all such modules or each module and associated code might be distributed between two or more devices as well.

As will be appreciated by one skilled in the art, various aspects may be embodied as a system, method or device program product. Accordingly, aspects may take the form of an entirely hardware embodiment or an embodiment including software that may all generally be referred to herein as a "circuit," "module" or "system." Furthermore, aspects may take the form of a device program product embodied in one or more device readable medium(s) having device readable program code embodied therewith.

It should be noted that the various functions described herein may be implemented using instructions or code stored on a device readable storage medium such as a non-signal storage device, where the instructions or code is/are executed by a processor or processors. A storage device may be, for example, an electronic, magnetic, optical, electromagnetic, infrared, or semiconductor system, apparatus, or device, or any suitable combination of the foregoing. More specific examples of a storage medium would include the following hardware: a portable computer diskette, a hard disk, a random access memory (RAM), a read-only memory (ROM), an erasable programmable read-only memory (EPROM or Flash memory), an optical fiber, a portable compact disc read-only memory (CD-ROM), an optical storage device, a magnetic storage device, or any suitable combination of the foregoing. In the context of this document, a storage device is a hardware device and is not a signal. Furthermore, "non-transitory" includes all media and devices except a signal.

Program code embodied on a storage medium may be transmitted using any appropriate medium, including but not limited to wireless, wireline, optical fiber cable, RF, etc., or any suitable combination of the foregoing.

Program code for carrying out operations may be written in any combination of one or more programming languages. The program code may execute entirely on a single device, partly on a single device, as a stand-alone software package, partly on single device and partly on another device, or entirely on the other device. In some cases, the devices may be connected through any type of connection or network, including a local area network (LAN) or a wide area network (WAN), or the connection may be made through other devices (for example, through the Internet using an Internet Service Provider), through wireless connections, e.g., short range wireless connections, near-field communication, or through a hard wire connection, such as over a USB connection.

Example embodiments are described herein with reference to the figures, which illustrate example methods, devices and program products according to various example embodiments. It will be understood that the actions and functionality may be implemented at least in part by program instructions or code. These program instructions or code may be provided to a processor of a device, such as a special purpose information handling device programmed with specific instructions or code as described herein, or other programmable data processing device to produce a machine, such that the instructions, which execute via a processor of the device, implement the functions/acts specified.

It is worth noting that while specific blocks and elements are used in the figures, and a particular ordering of blocks or elements has been illustrated, these are non-limiting examples. In certain contexts, two or more blocks or elements may be combined, a block or element may be split into two or more blocks or elements, or certain blocks or elements may be re-ordered or re-organized as appropriate, as the explicit illustrated examples are used only for descriptive purposes and are not to be construed as limiting.

As used herein, the singular "a" and "an" may be construed as including the plural "one or more" unless clearly indicated otherwise.

This disclosure has been presented for purposes of illustration and description but is not intended to be exhaustive or limiting. Many modifications and variations will be apparent to those of ordinary skill in the art. The example embodiments were chosen and described in order to explain principles and practical application, and to enable others of ordinary skill in the art to understand the disclosure for various embodiments with various modifications as are suited to the particular use contemplated.

Thus, although illustrative example embodiments have been described herein with reference to the accompanying figures, it is to be understood that this description is not limiting and that various other changes and modifications may be affected therein by one skilled in the art without departing from the scope or spirit of the disclosure.

## Claims

1. A system for automated hospital workforce load driven scheduling optimization, comprising:
a processor; and
a memory storing instructions executable by the processor to:
access scheduling information for a hospital unit comprising a number of staff available for working in the hospital unit and a staffing ratio for the hospital unit;
access a projected census value calculated reflecting current and future patient movement and progression within and between units;
calculate a number of projected workers needed for each hospital unit based on the projected census value and the staffing ratio for each hospital unit; and
output the number of projected workers needed for each hospital unit.

2. The system of claim 1, wherein the instructions are further executable by the processor to automatically identify workers from the number of staff available for working in each hospital unit.

3. The system of claim 2, wherein the instructions are further executable by the processor to automatically assign the identified workers to available beds based on the projected census value.

4. The system of claim 1, wherein the instructions are further executable by the processor to determine a mismatch between the number of projected workers needed for the hospital unit and the projected census value.

5. The system of claim 4, wherein:
the mismatch is a projected shortfall; and
the instructions are further executable by the processor to issue a notification regarding needed staffing.

6. The system of claim 5, wherein the instructions are further executable by the processor to access contact information for one or more of the identified workers and send a notification to the one or more identified workers regarding needed staffing.

7. The system of claim 4, wherein:
the mismatch is a projected over staffing; and
the instructions are further executable by the processor to issue a notification regarding needed staffing.

8. The system of claim 7, wherein the instructions are further executable by the processor to access contact information for one or more of the identified workers and send a notification to the one or more identified workers regarding needed staffing.

9. A method for automated hospital workforce load driven scheduling optimization, comprising:
accessing scheduling information, stored in an electronic memory device, for a hospital unit comprising a number of staff available for working in the hospital unit and a staffing ratio for the hospital unit;
accessing, using a processor, a projected census value calculated reflecting current and future patient movement and progression within and between units;
calculating, using a processor, a number of projected workers needed for the hospital unit based on the projected census value and the staffing ratio for the hospital unit; and
outputting, using a processor, the number of projected workers needed for the hospital unit.

10. A program product for automated hospital workforce load driven scheduling optimization, comprising:
a device readable storage unit having code stored therewith, the code being executable by a processor and comprising:
code that accesses scheduling information for a hospital unit comprising a number of staff available for working in the hospital unit and a staffing ratio for the hospital unit;
code that accesses a projected census value calculated reflecting current and future patient movement and progression within and between units;
code that calculates a number of projected workers needed for the hospital unit based on the projected census value and the staffing ratio for the hospital unit; and
code that outputs the number of projected workers needed for the hospital unit.
